Europäisches Patentamt

European Patent Office

Office européen des brevets

(11) Publication number: **0 233 581**
**A2**

## (12) EUROPEAN PATENT APPLICATION

(21) Application number: **87101857.8**

(22) Date of filing: **11.02.87**

(51) Int. Cl.⁴: **C12P 13/06** , C12P 13/08 ,
C12N 15/00 , C12N 1/20 ,
//(C12N1/20,C12R1:15,1:13)

The applicant has filed a statement in accordance with Rule 28 (4) EPC (issue of a sample only to an expert). Accession number(s) of the deposit(s): Ferm BP - 973, Ferm BP - 1052, Ferm BP - 1053, Ferm BP - 158, Ferm BP - 455.

(30) Priority: **12.02.86 JP 28271/86**

(43) Date of publication of application:
**26.08.87 Bulletin 87/35**

(84) Designated Contracting States:
**DE FR GB**

(71) Applicant: **KYOWA HAKKO KOGYO CO., LTD.
Ohtemachi Bldg., 6-1 Ohtemachi I-chome
Chiyoda-ku Tokyo 100(JP)**

(72) Inventor: **Katsumata, Ryoichi c/o Kyowa
Hakko Kogyo Co., Ltd.
6-1, Ohtemachi Itchome
Chiyoda-ku Tokyo(JP)**
Inventor: **Mizukami, Toru c/o Kyowa Hakko
Kogyo Co., Ltd.
6-1, Ohtemachi Itchome
Chiyoda-ku Tokyo(JP)**
Inventor: **Kino, Kuniki c/o Kyowa Hakko Kogyo
Co., Ltd.
6-1, Ohtemachi Itchome
Chiyoda-ku Tokyo(JP)**
Inventor: **Kikuchi, Yasuhiro c/o Kyowa Hakko
Kogyo Co., Ltd.
6-1, Ohtemachi Itchome
Chiyoda-ku Tokyo(JP)**
Inventor: **Hotta, Keiko c/o Kyowa Hakko Kogyo
Co., Ltd.
6-1, Ohtemachi Itchome
Chiyoda-ku Tokyo(JP)**

(74) Representative: **Vossius & Partner
Siebertstrasse 4 P.O. Box 86 07 67
D-8000 München 86(DE)**

(54) Process for producing L-threonine or L-isoleucine.

(57) Productivity of L-threonine or L-isoleucine is conferred or increased in the microorganisms of the genus Corynebacterium or Brevibacterium by having microorganisms of the genus Corynebacterium or Brevibacterium carry a recombinant DNA bearing all the genetic information of HD, HK and TS responsible for the threonine biosynthesis originating from microorganisms belonging to the genus Corynebacterium or Brevibacterium or plural recombinant DNAs bearing the genetic information separately. The transformant produces enhanced quantities of L-threonine or L-isoleucine upon culturing in a medium.

## FIG. 1

# PROCESS FOR PRODUCING L-THREONINE OR L-ISOLEUCINE

## Background of the Invention

The present invention relates to a process for producing L-threonine or L-isoleucine by recombinant DNA technology.

More specifically, the present invention relates to a process for producing L-threonine or L-isoleucine, which comprises culturing a microorganism belonging to the genus Corynebacterium or Brevibacterium which harbors a recombinant DNA containing a DNA fragment bearing genetic information responsible for the synthesis of homoserine dehydrogenase (hereinafter referred to as HD), homoserine kinase (hereinafter referred to as HK) and threonine synthase (hereinafter referred to as TS) originating from microorganisms belonging to the genus Corynebacterium or Brevibacterium in a culture medium, accumulating L-threonine or L-isoleucine in the culture broth and recovering L-threonine or L-isoleucine therefrom. Thus, the present invention provides an effective means for producing L-threonine and L-isoleucine which are particularly useful in medicines, animal feed and food.

In the process for producing L-threonine and L-isoleucine by fermentation with microorganisms of the genus Corynebacterium or Brevibacterium, it has heretofore been known to use mutant strains derived from the wild type strains of such genus. Known L-threonine-and L-isoleucine-producing mutant strains are those having an amino acid-requiring mutation and/or an amino acid analog-resistant mutation as disclosed, for example, in Japanese Published Unexamined Patent Application No. 19087/72, Japanese Published Examined Patent Application No. 32070/79, etc.

Apart from strains obtained by endowment of these mutations, processes for producing L-threonine or L-isoleucine by using strains constructed by recombinant DNA technology have also been known. For example, microorganisms of the genus Corynebacterium or Brevibacterium carrying a recombinant DNA that contains genes coding for enzymes involved in threonine biosynthesis isolated from Escherichia coli are used for a fermentative process for producing L-threonine of L-isoleucine (Japanese Published Unexamined Patent Application Nos. 126789/83 and 30693/85). Furthermore, a process for producing L-threonine or L-isoleucine with microorganisms of the genus Corynebacterium or Brevibacterium carrying a recombinant DNA that contains an HD gene isolated from a strain of the genus Brevibacterium is disclosed in Japanese Published Unexamined Patent Application No. 12995/85. Still furthermore, a process for producing L-threonine or L-isoleucine with microorganisms of the genus Corynebacterium or Brevibacterium carrying a recombinant DNA that contains an HD gene and an HK gene isolated from a strain of the genus Corynebacterium is also disclosed in Japanese Patent Application No. 19801/86.

With the recent increase in the demand for L-threonine and L-isoleucine, improved processes for the industrial production thereof are desired. The present inventor has further studied to the end that the L-threonine-and L-isoleucine-producing ability of microorganisms of the genus Corynebacterium or Brevibacterium is increased by recombinant DNA technology.

## Summary of the Invention

In accordance with the present invention, L-threonine or L-isoleucine can be produced in high yield by culturing a microorganism belonging to the genus Corynebacterium or Brevibacterium which harbors a recombinant DNA comprising a DNA fragment bearing genetic information responsible for the synthesis of HD, HK and TS isolated from a microorganism of the genus Corynebacterium or Brevibacterium, and a vector plasmid in a culture medium, accumulating L-threonine or L-isoleucine in the culture broth and recovering L-threc 'ne or L-isoleucine therefrom.

It has been found that the ability of microorganisms belonging to the genus Corynebacterium or Brevibacterium to produce L-threonine and L-isoleucine can considerably be increased by having the microorganisms carry a recombinant DNA that contains a DNA fragment bearing genetic information of HD, HK and TS isolated from microorganisms belonging to the genus Corynebacterium or Brevibacterium. It has first been found that the productivity of L-threonine and L-isoleucine can remarkably increase by amplification effect of the three genes.

Brief Description of the Drawing

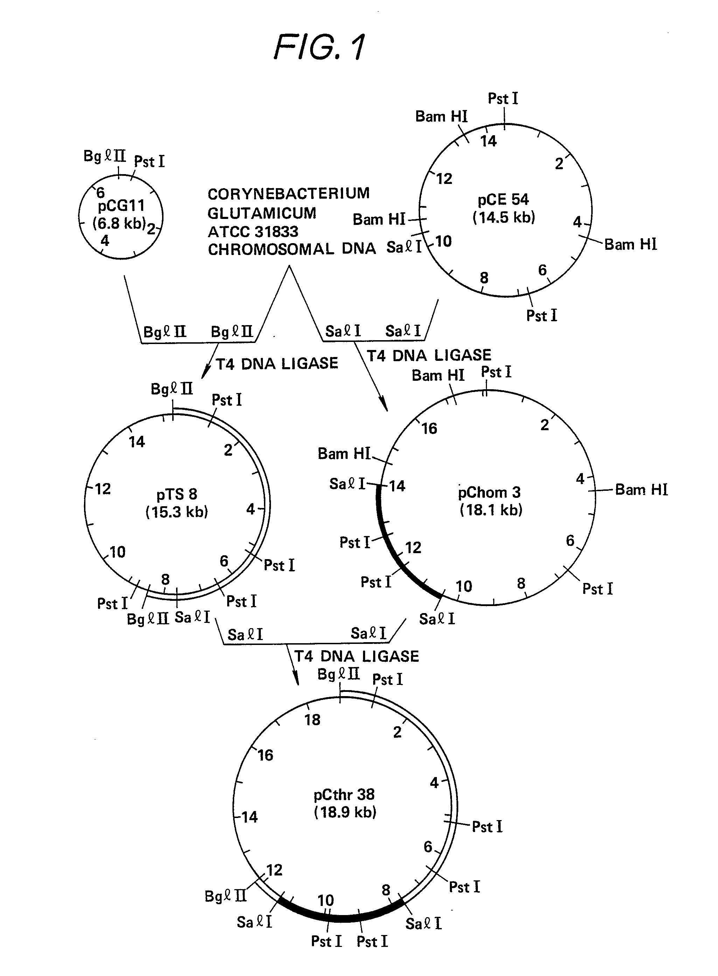

Fig. 1 illustrates the cleavage maps of pChom3, pTS8 and pCthr38 for the restriction enzymes SalI, BgIII, BamHI and PstI, and the steps for constructing the plasmids, where the size of the plasmids is given in kilobases (kb). The chromosomal DNA fragment indicated by the heavy line of pChom3 contains both HD and HK genes, chromosomal DNA fragment indicated by the double solid lines of pTS8 contains TS gene, and the chromosomal DNA fragment indicated by the heavy line and the double solid lines of pCthr38 contains HD, HK and TS genes, respectively.

Description of the Invention

All the microorganisms known as glutamic acid-producing coryneform bacteria can be used as a host microorganism belonging to the genus Corynebacterium or Brevibacterium . The following strains are exemplary of those particularly suitable.
Corynebacterium glutamicum ATCC31833
Corynebacterium glutamicum ATCC13032
Corynebacterium acetoacidophilum ATCC13870
Corynebacterium herculis ATCC13868
Corynebacterium lilium ATCC15990
Brevibacterium divaricatum ATCC14020
Brevibacterium flavum ATCC14067
Brevibacterium immariophilum ATCC14068
Brevibacterium lactofermentum ATCC13869
Brevibacterium thiogenitalis ATCC19240

As a host microorganism, L-threonine-or L-isoleucine-non-producing strains can be used, but L-threonine-, L-isoleucine-or L-lysine-producing strains are preferably used. The L-threonine-, L-isoleucine-or L-lysine-producing strains can be obtained by conventional mutations such as a mutation in amino acid-requirement, a mutation in resistance to analogs or a combination of these mutations [Nakayama, K. In G. Reed (ed.), Prescott and Dunn's Industrial Microbiology, 4th ed. pp.748 -801, (1982), AVI Publishing Co., Westport, Conn.].

In case of using L-lysine-producing microorganisms as a host, the L-lysine production is converted to L-threonine or L-isoleucine production, and strains capable of accumulating a considerable amount of L-threonine or L-isoleucine can be obtained. It has first been found that metabolic conversion to L-threonine or L-isoleucine is carried out in L-lysine-producing strains.

Any microorganism can be used in the present invention as chromosomal DNA of any microorganism available for a supply source of HD, HK and TS genes, as long as they are glutamic acid-producing coryneform bacteria and also have HD, HK and TS activities. For example, wild type strains of the genus Corynebacterium or Brevibacterium, or L-threonine-or L-isoleucine-producing mutant strains derived therefrom can be used. Chromosomal DNA of these strains can be isolated by treating the cells that have been treated with penicillin during the culturing with lysozyme and a surfactant for bacteriolysis, then removing proteins therefrom according to a conventional method, and then conducting ethanol precipitation thereof, as shown in Japanese Published Unexamined Patent Application No. 126789/83.

Any vector that is autonomously replicable in the microorganisms of the genus Corynebacterium or Brevibacterium is available for integration of a DNA fragment containing HD, HK and TS genes, and, for example, plasmids such as pCG1 (Japanese Published Unexamined Patent Application No. 134500/82), pCG2 (Japanese Published Unexamined Patent Application No. 35197/83), pCG4 and pCG11 (Japanese Published Unexamined Patent Application No. 183799/82), pCE54 and pCB101 (Japanese Published Unexamined Patent Application No. 105999/83), pCE51 (Japanese Published Unexamined Patent Application No. 34197/85), pCE52 and pCE53 [Mol. Gen. Genet. 196, 175 (1984)], etc. can be used. Plasmid vectors are isolated and purified as CCC-DNA by treating the cells with lysozyme and surfactant for bacteriolysis, preparing the cleared lysates therefrom, precipitating DNAs with polyethyleneglycol, and subjecting DNAs to density gradient centrifugation with cesium chloride-ethidium bromide as disclosed in Japanese Published Unexamined Patent Application Nos. 134500/82 and 186489/82.

A recombinant DNA containing a DNA fragment containing a TS gene can be constructed together with a mixture of various recombinants according to a conventional method, for example, by digesting chromosomal DNA and vector plasmid with a restriction enzyme, followed by treatment with a DNA ligase or by digesting chromosomal DNA and vector plasmid with a restriction enzyme, followed by treatment of cleaved terminals with terminal transferase, DNA polymerase, etc., and successive treatment with a DNA ligase, etc. [Methods in Enzymology 68 (1979)].

A recombinant DNA containing a TS gene can be obtained by transforming TS-deficient mutant strains having a threonine-requirement and being incapable of secreting homoserine, derived from a strain of the genus Corynebacterium or Brevibacterium according to ordinary mutation operations with the said mixture of recombinants, and then selecting a threonine-non-requiring transformant. Transformation of strains of the genus Corynebacterium or Brevibacterium is carried out according to a method using a protoplast as described in the following Example and Japanese Published Unexamined Patent Application Nos. 186489/82 and 186492/82.

Likewise, a recombinant DNA containing a DNA fragment containing both HD and HK genes can be obtained (Japanese Patent Application No. 19801/86). That is, HD-deficient mutant strains having homoserine-requirement or HK-deficient mutant strains having threonine-requirement and capable of secreting homoserine, derived from the strains of the genus Corynebacterium or Brevibacterium are transformed with a mixture of recombinants of chromosomal DNA and a vector plasmid, and recombinant plasmids containing HD or HK gene can be obtained from the transformants having homoserine-non-requirement or threonine-non-requirement. A recombinant plasmid containing both HD and HK genes can be obtained by selecting one in which both HD-deficiency and HK-deficiency have been restored.

A recombinant DNA containing TS, HD and HK genes can be obtained by further recombinantion of the thus obtained DNA fragment containing a TS gene with the thus obtained DNA fragment containing both HD and HK genes. The three genes can be amplified by introducing the recombinant DNA into host microorganisms. The three genes can also be amplified, if they are contained in independent vector plasmids capable of coexisting in one cell one by one and these plasmids are simultaneously contained in a host microorganism. In both cases, the increased productivity of L-threonine or L-isoleucine can be achieved.

When chromosomal DNA of the wild type strains of the genus Corynebacterium or Brevibacterium is used as a supply source in the foregoing processes, a recombinant DNA containing HD, HK and TS genes of wild type can be obtained, and if only such a recombinant DNA is allowed to be contained in strains of the genus Corynebacterium or Brevibacterium, the productivity of L-threonine or L-isoleucine can be increased. However, it is known that in the threonine biosynthesis of microorganisms of the genus Corynebacterium or Brevibacterium, HD is subjected to feedback inhibition by threonine to control the threonine synthesis [Agric. Biol. Chem. 38 (5), 993 (1974)], and thus the productivity of L-threonine and L-isoleucine can be still more increased by using a recombinant plasmid having a gene coding for mutant HD released from the inhibition.

Such a recombinant plasmid containing the mutant HD gene can be obtained by using chromosomal DNA of a mutant strain resistant to threonine analogs (for example, $\alpha$-amino-$\beta$-hydroxyvaleric acid), in which HD has been released from the inhibition by threonine as a supply source in the same method as that used for constructing a recombinant plasmid containing the gene of wild type, as disclosed in Agric. Biol. Chem., 38 (5), 993 (1974). The recombinant plasmid containing a gene coding for HD released from the inhibition by threonine can also be prepared by subjecting microorganisms of the genus Corynebacterium or Brevibacterium carrying a recombinant plasmid that contains an HD gene of wild type to a conventional mutation treatment, and thereby endowing a resistance to threonine analogs therewith.

The recombinant plasmid containing HD, HK and TS genes of wild type or mutant type can be introduced into microorganisms of the genus Corynebacterium or Brevibacterium by transformation method using such a protoplast as mentioned above.

Production of L-threonine or L-isoleucine by strains carrying these recombinant plasmids is carried out according to conventional fermentative methods for producing L-threonine or L-isoleucine. That is, by culturing the transformant in an ordinary medium containing a carbon source, a nitrogen source, inorganic compounds, amino acids, vitamins, etc. under aerobic conditions while adjusting the temperature, pH, etc., L-threonine or L-isoleucine is accumulated in the culture broth, and may be recovered therefrom by standard procedures.

4

As the carbon source, carbohydrates such as glucose, glycerol, fructose, sucrose, maltose, mannose, starch, starch hydrolyzate, molasses, etc.; polyalcohols; and various organic acids such as pyruvic acid, fumaric acid, lactic acid, acetic acid, etc. can be used. Furthermore, hydrocarbons, alcohols, etc. can be used depending upon the assimilability of the microorganism to be used. Particularly, cane molasses is preferably used.

As the nitrogen source, ammonia, various inorganic and organic ammonium salts such as ammonium chloride, ammonium sulfate, ammonium carbonate, ammonium acetate, etc., urea and other nitrogen-containing materials as well as various nitrogen-containing organic materials such as peptone, NZ-amine, meat extract, yeast extract, corn steep liquor, casein hydrolyzate, fish meal or its digested product, chrysalis hydrolyzate and the like can be used.

As inorganic compounds, potassium dihydrogenphosphate, dipotassium hydrogenphosphate, ammonium sulfate, ammonium chloride, magnesium sulfate, sodium chloride, ferrous sulfate, manganese sulfate, calcium carbonate and the like are appropriate. Naturally, if vitamins, amino acids, etc. required for the growth of the microorganism are supplied by other medium components described above, it is not necessary to add these specific nutrients separately to the medium.

Culturing is carried out under aerobic conditions, for example, by shaking culture or by aeration-stirring culture. The preferred culturing temperature is generally 20 to 40°C; and the pH of the culture medium is preferably maintained around the neutrality. Usually, after culturing for 1 to 5 days under these conditions, recoverable quantities of L-threonine and/or L-isoleucine are accumulated in the culture broth. The cells are removed from the culture broth and then L-threonine and/or L-isoleucine accumulated is recovered from the culture liquor according to known procedures, for example, by activated carbon treatment or by ion exchange resin treatment.

L-threonine and/or L-isoleucine can thus be produced in high yield using a microorganism of the genus Corynebacterium or Brevibacterium carrying a recombinant plasmid that contains HD, HK and TS genes originating from microorganisms belonging to the genus Corynebacterium or Brevibacterium in this manner.

Japanese Published Unexamined Patent Application No. 126789/83 corresponds to European Publication No. 88166. Japanese Published Unexamined Patent Application No. 30693/85 corresponds to European Publication No. 136359. Japanese Published Unexamined Patent Application No. 12995/85 corresponds to European Publication No. 131171, US Patent No. 4601983. Japanese Patent Application No. 19801/86 corresponds to European Publication No. 197335. Japanese Published Unexamined Patent Application No. 134500/82 corresponds to European Publication No. 58889, US Patent No. 4617267. Japanese Published Unexamined Patent Application No. 35197/83 corresponds to European Publication No. 73062, US Patent No. 4489160. Japanese Published Unexamined Patent Application No. 183799/82 corresponds to European Publication No. 63763, US Patent No. 4500640. Japanese Published Unexamined Patent Application No. 105999/83 corresponds to European Publication No. 82485. Japanese Published Unexamined Patent Application No. 34197/85 corresponds to European Publication No. 13659. Japanese Published Unexamined Patent Application No. 186489/82 corresponds to European Publication No. 64680. Japanese Published Unexamined Patent Application No. 186492/82 corresponds to European Publication No. 63764.

A certain specific embodiment of the present invention is illustrated by the following representative example.

Example

(1) Preparation of chromosomal DNA of Corynebacterium glutamicum ATCC31833 and vectors pCG11 and pCE54:

A seed culture of Corynebacterium glutamicum ATCC31833 grown in NB medium (a medium containing 20 g of bouillon powder and 5 g of yeast extract in 1 $\ell$ of deionized water, and adjusted to pH 7.2) was inoculated into 400 m$\ell$ of semi-synthetic medium SSM [a medium containing 20 g of glucose, 10 g of $(NH_4)_2SO_4$, 3 g of urea, 1 g of yeast extract, 1 g of $KH_2PO_4$, 0.4 g of $MgCl_2 \cdot 6H_2O$, 10 mg of $FeSO_4 \cdot 7H_2O$, 0.2 mg of $MnSO_4 \cdot 4\text{-}6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 $\mu$g of biotin and 1 mg of thiamine hydrochloride in 1 $\ell$ of water, and adjusted to pH 7.2] and was subjected to shaking culture at 30°C. The optical density at 660 nm (OD) was determined with a Tokyo Koden colorimeter and penicillin G was added to a final concentration of 0.5 units/m$\ell$ when the OD reached 0.2. Culturing was continued until the OD reached 0.6.

Then, the cells were collected from the culture broth and washed with TES buffer solution [0.03 M Tris (hydroxymethyl)amino-methane (hereinafter referred to as Tris), 0.005 M EDTA (disodium ethylenediaminetetraacetate) and 0.05 M NaCl, pH 8.0]. The washed cells were suspended in 10 mℓ of a lysozyme solution (25% sucrose, 0.1 M NaCl, 0.05 M Tris and 0.8 mg/mℓ lysozyme, pH 8.0; the same lysozyme solution was hereinafter used), and reacted at 37°C for 4 hours. The cells treated with lysozyme were collected and chromosomal DNA was isolated from the cells according to the method of Saito-Miura - [Saito, H. and Miura, K.: Biochim. Biophys. Acta, 72, 619 (1963)].

pCG11 (Japanese Published Unexamined Patent Application No. 183799/82) used as a vector is a plasmid constructed by combining plasmid pCG1 of Corynebacterium glutamicum (Japanese Published Unexamined Patent Application No. 134500/82) with a DNA fragment containing a gene coding for spectinomycin/streptomycin-resistant character originating from plasmid pCG4 of Corynebacterium glutamicum (Japanese Published Unexamined Patent Application No. 183799/82), and more specifically a plasmid constructed by combining BglII-cleaved fragment of pCG1 with BamHI-cleaved DNA fragment containing a gene coding for spectinomycin/streptomycin-resistant character of pCG4 through the same cohesive ends of these fragments.

Vector pCE54 (Japanese Published Unexamined Patent Application No. 105999/83) is a plasmid constructed by combining the plasmid pCG2 (Japanese Published Unexamined Patent Application No. 35197/83) with plasmid pGA22 of Escherichia coli [J. Bacteriol. 140, 400 (1979)] at single Pst I-cleavage site on the individual plasmids.

These vector plasmids pCG11 and pCE54 were isolated from cultured cells of Corynebacterium glutamicum ATCC39019 (the strain having a lysozyme-sensitive mutation, derived from ATCC31833) containing each of the plasmids in the following manner.

The strain was subjected to shaking culture in 400 mℓ of NB medium at 30°C, and culturing was continued until the OD reached about 0.7. The cells were collected, washed with TES buffer solution, then suspended in 10 mℓ of a lysozyme solution and subjected to reaction at 37°C for 2 hours. Then, 2.4 mℓ of 5 M NaCl, 0.6 mℓ of 0.5 M EDTA (pH 8.5), and 4.4 mℓ of a solution consisting of 4% sodium laurylsulfate and 0.7 M NaCl were added in order to the reaction solution, and the mixture was gently stirred and placed on ice water for 15 hours. The bacteriolytic product was transferred into a centrifuge tube, and subjected to centrifugation at 69,400 x g at 4°C for 60 minutes to recover a supernatant. Polyethyleneglycol (PEG) 6,000 (product of Nakarai Kagaku Yakuhin Co.) in an amount corresponding to 10% by weight was added thereto and the mixture was gently stirred for lysis. The solution was placed on ice water, and after 10 hours, centrifuged at 1,500 x g for 10 minutes to recover pellets. Then, 5 mℓ of TES buffer solution was added to gently redissolve the pellets, and then 2.0 mℓ of 1.5 mg/mℓ ethidium bromide was added thereto. Cesium chloride was further added thereto and gently dissolved to adjust the density of the solution to 1.580.

The solution was subjected to ultracentrifugation at 105,000 x g at 18°C for 48 hours, and a band at a high density level at the lower position of the centrifuge tube detected under ultraviolet irradiation was recovered from the side of the centrifuge tube through a syringe, whereby pCG11 or pCE54 plasmid DNA was isolated therefrom. To remove ethidium bromide by extraction, the fraction was treated 5 times with an equal volume of isopropyl alcohol solution consisting of 90% by volume isopropyl alcohol and 10% by volume TES buffer solution, and further containing a saturation amount of cesium chloride, and then dialyzed against TES buffer solution.

(2) Cloning of DNA fragment containing a TS gene:

Six units of restriction enzyme BglII (product of Takara Shuzo Co.) was added to 60 μℓ of a reaction solution for restriction enzyme BglII (10 mM Tris, 6 mM MgCl₂, 100 mM NaCl, 7 mM 2-mercaptoethanol, pH 7.5) containing 3 μg of pCG11 plasmid DNA prepared above, and the mixture was subjected to reaction at 37°C for 60 minutes. Then, the reaction was stopped by heating the mixture at 65°C for 10 minutes.

Separately, 4 units of BglII was added to 140 μℓ of reaction solution for BglII containing 8 μg of chromosomal DNA of Corynebacterium glutamicum ATCC31833, and the mixture was subjected to reaction at 37°C for 60 minutes. The reaction was stopped by heating the mixture at 65°C for 10 minutes.

Both reaction solutions were mixed together, and 40 μℓ of a buffer solution for T4 ligase (660 mM Tris, 66 mM MgCl₂ and 100 mM dithiothreitol, pH 7.6) at a 10-fold concentration, 40 μ of 5 mM ATP, 0.3 μℓ of T4 ligase (1 unit/μℓ, product of Takara Shuzo Co.) and 120 μℓ of water were added thereto. The mixture was subjected to reaction at 12°C for 16 hours.

The thus obtained ligase reaction mixture was used for transformation of K60 strain (the strain which is a TS-deficient mutant strain having threonine-requirement and incapable of secreting homoserine), derived from Corynebacterium glutamicum ATCC31833. The K60 strain was deposited as FERM BP-973 with the Fermentation Research Institute (FRI), Agency of Industrial Science and Technology on January 24, 1986.

Protoplasts prepared according to the following manner were used in the transformation. A seed culture of K60 strain was inoculated in SSM medium containing 200 $\mu$g/m$l$ threonine, and cultured with shaking at 30°C. When the OD reached 0.2, penicillin G was added to a final concentration of 0.5 units/m$l$. Culturing was continued and when the OD reached 0.6, the cells were collected and suspended in a solution (pH 7.6) containing 1 mg/m$l$ lysozyme in RCGP medium comprising 5 g of glucose, 5 g of casamino acid, 2.5 g of yeast extract, 3.5 g of $K_2HPO_4$, 1.5 g of $KH_2PO_4$, 0.41 g of $MgCl_2$•$6H_2O$, 10 mg of $FeSO_4$•$7H_2O$, 2 mg of $MnSO_4$•$4$-$6H_2O$, 0.9 mg of $ZnSO_4$•$7H_2O$, 0.04 mg of $(NH_4)_6Mo_7O_{24}$•$4H_2O$, 30 $\mu$g of biotin, 2 mg of thiamine hydrochloride, 135 g of disodium succinate, and 30 g of polyvinylpyrrolidone having a molecular weight of 10,000 in 1 $l$ of water to make up about $10^9$ cells/m$l$. The suspension was transferred to an L-tube, and subjected to gentle shaking culture at 30°C for 5 hours to induce protoplasts. Then, 0.5 m$l$ of the protoplast suspension was taken into a small test tube, and centrifuged at 2,500 x g for 5 minutes. The protoplasts were resuspended in 1 m$l$ of TSMC buffer solution (10 mM $MgCl_2$, 30 mM $CaCl_2$, 50 mM Tris and 400 mM sucrose at pH 7.5), washed by centrifugation, and then resuspended in 0.1 m$l$ of TSMC buffer solution. Then, 100 $\mu l$ of a 1:1 mixture of TSMC buffer solution at a two-fold concentration and the said ligase reaction solution was added to the protoplast suspension and mixed. Then, 0.8 m$l$ of TSMC buffer solution containing 20% PEG 6,000 was added thereto and mixed. Three minutes thereafter, 2 m$l$ of RCGP medium (pH 7.2) was added thereto, and the mixture was centrifuged at 2,500 x g for 5 minutes to remove a supernatant. The precipitated protoplasts were suspended in 1 m$l$ of RCGP medium, and 0.2 m$l$ of the suspension was spread onto RCGP agar medium (RCGP medium containing 1.4% agar at pH 7.2) containing 400 $\mu$g/m$l$ spectinomycin and cultured at 30°C for 7 days.

Colonies grown on the agar medium were collected by scraping, washed twice with physiological saline solution by centrifugation, and then suspended in 1 m$l$ of physiological saline solution. The cells were respread onto minimal agar medium M1 [a medium containing 10 g of glucose, 1 g of $NH_4H_2PO_4$, 0.2 g of KCl, 0.2 g of $MgSO_4$•$7H_2O$, 10 mg of $FeSO_4$•$7H_2O$, 0.2 mg of $MnSO_4$•$4$-$6H_2O$, 0.9 mg of $ZnSO_4$•$7H_2O$, 0.4 mg of $CuSO_4$•$5H_2O$, 0.09 mg of $Na_2B_4O_7$•$10H_2O$, 0.04 mg of $(NH_4)_6Mo_7O_{24}$•$4H_2O$, 50 $\mu$g of biotin, 2.5 mg of p-aminobenzoic acid, 1 mg of thiamine hydrochloride and 16 g of agar in 1 $l$ of water, and adjusted to pH 7.2] containing 100 $\mu$g/m$l$ spectinomycin and cultured at 30°C for 3 days, and transformants having threonine-non-requirement and resistance to spectinomycin were selected.

The transformants were cultured in SSM medium and treated with penicillin G in the same manner as described above, and plasmid DNAs were isolated from the cultured cells in the same manner as used to isolate pCG11 in the foregoing section (1). A plasmid obtained from one of the transformants and named "pTS8" was found as a result of analysis by digestion with various restriction enzymes and agarose gel electrophoresis to have such a structure that BglII DNA fragment of 8.5 kb was inserted at the single BglII-cleaved site of pCG11. The BglII-cleaved fragment had three PstI-cleavage sites and one SalI-cleavage site as illustrated in Fig. 1.

(3) Cloning of DNA fragment containing an HD gene and an HK gene:

Cloning of a DNA fragment containing HD and HK genes was carried out in the same manner as used in cloning of TS gene. As a vector, pCE54 plasmid prepared in the foregoing section (1) was used, and as a recipient for cloning and HD-deficient strain was used.

Ten units of SalI (product of Takara Shuzo Co.) was added to 200 $\mu l$ of a reaction solution for SalI (10 mM Tris, 6 mM $MgCl_2$ and 200 mM NaCl, pH 7.5) containing 3 $\mu$g of pCE54 plasmid DNA and 8 $\mu$g of chromosomal DNA of ATCC31833, and subjected to reaction at 37°C for 60 minutes. Then the reaction was stopped by heating the reaction mixture at 65°C for 10 minutes.

Then, 40 $\mu l$ of a T4 ligase buffer solution at a 10-fold concentration, 40 $\mu l$ of 5 mM ATP, 0.3 $\mu l$ of T4 ligase (1 unit/$\mu l$) and 120 $\mu l$ of water were added to the reaction mixture and the mixture was subjected to reaction at 12°C for 16 hours.

The thus obtained ligase reaction mixture was used for transforming K53 strain (the strain which is an HD-deficient mutant strain having homoserine-requirement and leucine-requirement, and deposited in the FRI as FERM BP-1052 on May 23, 1985), derived from a lysozyme-sensitive mutant strain originating from Corynebacterium glutamicum ATCC31833. That is, a seed culture of K53 strain was inoculated in NB medium and cultured by shaking at 30°C, and the cells were collected at the time when the OD reached 0.6. The cells were converted to protoplasts in the same manner as used in the foregoing section (2), and the protoplast suspension and the ligase reaction mixture were treated with PEG and spread onto RCGP agar medium containing 300 $\mu$g/m$l$ kanamycin and cultured.

Colonies grown on the culture medium were collected by scraping, washed with physiological saline solution by centrifugation, and respread onto minimal agar medium M1 containing 20 $\mu$g/m$l$ kanamycin and 50 $\mu$g/m$l$ leucine to obtain transformants having homoserine-non-requirement and resistance to kanamycin.

Plasmid DNA was isolated from one of the transformants, in the same manner as used in the foregoing section (1), and named pChom3. The plasmid had such a structure that SalI DNA fragment of 3.6 kb was inserted at the single SalI-cleaved site of pCE54. On SalI DNA fragment of 3.6 kb, there are two PstI-cleavage sites as illustrated in Fig. 1.

The presence of both HD and HK genes on pChom3 plasmid was confirmed in the following manner. Protoplasts of K53 strain and K54 strain (HK-deficient strain having threonine-requirement and homoserine-productivity, deposited as FERM BP-1053 in the FRI on May 23, 1985) were transformed with pChom3 DNA in the same manner as described above. Protoplasts of K54 strain were prepared from the penicillin-treated cells in the same manner as used in the foregoing section (2). Transformants selected on the basis of the resistance to kanamycin had homoserine-non-requirement or threonine-non-requirement at the same time, and the plasmid isolated from these transformants had the same structure as that of pChom3. It is seen therefrom that both HD and HK genes were cloned on pChom3.

Similar tests were conducted for pTS8 plasmid on K53 and K54 strains, and for pChom3 plasmid on K60 strain. The requirements were not complemented in either cases. Thus, it was recognized that there was no HD and HK genes on the pTS8 plasmid and that there was no TS gene on the pChom3 plasmid.

(4) Preparation of recombinant plasmid simultaneously containing HD, HK and TS genes:

Six units of SalI was added to 200 $\mu l$ of a reaction solution for SalI containing 3 $\mu$g of pTS8 plasmid DNA and 3 $\mu$g of pChom3 plasmid DNA, and subjected to reaction at 37°C for 60 minutes, and then the reaction was stopped by heating the mixture at 65°C. Then, 40 $\mu l$ of a buffer solution for T4 ligase at a 10-fold concentration, 40 $\mu l$ of 5 mM ATP, 0.3 units of T4 ligase and 120 $\mu l$ of water were added thereto, and the mixture was subjected to reaction at 12°C for 16 hours.

Corynebacterium glutamicum K53 strain was transformed with the resulting ligase reaction mixture. The transformants having a resistance to spectinomycin was spread onto NB agar medium containing 20 $\mu$g/m$l$ kanamycin and also onto minimal agar medium M1 containing 50 $\mu$g/m$l$ leucine and 100 $\mu$g/m$l$ spectinomycin. A plasmid was isolated and purified from one of the transformants having resistance to spectinomycin, kanamycin-sensitivity and homoserine-non-requirement. The plasmid had such a structure that SalI DNA fragment of 3.6 kb originating from pChom3 was inserted at the single SalI-cleaved site of pTS8 plasmid (see Fig. 1). The plasmid was named pCthr38.

HD-deficient mutant strain K53, HK-deficient mutant strain K54 and TS-deficient mutant strain K60 were transformed with pCthr38, and it was found that the transformant having resistance to spectinomycin did not require homoserine or threonine at the same time. It is seen therefrom that pCthr38 plasmid contains HD, HK and TS genes.

(5) Production of threonine by strains carrying pChom3, pCthr38 or pChom3 and pTS8:

Corynebacterium glutamicum ATCC31833, Corynebacterium herculis ATCC13868, Brevibacterium lactofermentum ATCC13869 and a lysine-producing mutant strain Brevibacterium flavum ATCC21475 were transformed with pChom3 and pCthr38. Protoplasts were prepared in the same manner as used in the foregoing section (2) where protoplasts of K60 strain were prepared. That is, the protoplasts were prepared by adding the penicillin G (0.45 units/m$l$) to the cells during the culturing on SSM medium, and treating the cells with lysozyme. Protoplasts were transformed with 1 $\mu$g of plasmid DNAs in the same manner as described above, and transformants having resistance to kanamycin or resistance to spectinomycin were selected on RCGP agar medium. A plasmid was isolated from the transformants in the same manner as used in the foregoing section (2) where a plasmid was isolated from pTS8-containing transformant of K60 strain, and it was recognized by digestion analysis with various restriction enzymes that the transformant contained pChom3 or pCthr38.

8

The thus obtained pChom3-carrying transformant was further transformed with pTS8 in the same manner as described above. Plasmids were isolated from the transformants selected on the basis of resistance to kanamycin and to spectinomycin in the same manner as described above, and it was recognized by digestion analysis with various restriction enzymes that the transformants contained both pChom3 and pTS8.

Threonine production tests of the transformants and the parent strains thereof were carried out as follows. First, 0.5 mℓ of a seed culture obtained by shaking culture in NB medium at 30°C for 16 hours was put into a test tube containing 5 mℓ of a production medium [a medium containing 100 g of glucose, 20 g of $(NH_4)_2SO_4$, 0.5 g of $KH_2PO_4$, 0.5 g of $K_2HPO_4$, 1 g of $MgSO_4•7H_2O$, 10 mg of $FeSO_4•7H_2O$, 10 mg of $MnSO_4•4-6H_2O$, 100 µg of biotin and 20 g of calcium carbonate in 1 ℓ of water, and adjusted to pH 7.2] and cultured with shaking at 30°C for 72 hours. After culturing, culture filtrate was subjected to paper chromatography and the amount of L-threonine produced was determined by colorimetry using ninhydrin color development. Results are shown in Table 1.

## Table 1

| Strain | Amount of threonine produced (g/ℓ) |
|---|---|
| Corynebacterium glutamicum ATCC31833 | 0 |
| "               ATCC31833/pChom3 | 0.4 |
| "               ATCC31833/pCthr38 | 2.0 |
| "               ATCC31833/pChom3, pTS8 | 1.8 |
| Corynebacterium herculis ATCC13868 | 0 |
| "               ATCC13868/pChom3 | 0.6 |
| "               ATCC13868/pCthr38 | 2.5 |
| "               ATCC13868/pChom3, pTS8 | 2.4 |
| Brevibacterium lactofermentum ATCC13869 | 0 |
| "               ATCC13869/pChom3 | 0.3 |
| "               ATCC13869/pCthr38 | 1.8 |
| "               ATCC13869/pChom3, pTS8 | 1.7 |
| Brevibacterium flavum ATCC21475 | 0 |
| "               ATCC21475/pChom3 | 0.3 |
| "               ATCC21475/pCthr38 | 2.0 |
| "               ATCC21475/pChom3, pTS8 | 1.9 |

(6) Production of isoleucine by strains containing pChom3, pTS8, pCthr38 or pChom3 and pTS8:

Brevibacterium flavum ATCC14067, Corynebacterium glutamicum K40 which is isoleucine-producing mutant strain (FERM BP-455, deposited with the FRI on July 21, 1983), and Corynebacterium glutamicum H-3149 which is lysine-producing mutant strain (FERM BP-158, deposited with the FRI on June 14, 1982) were transformed in the same manner as used in the foregoing section (5) with pChom3, pTS8 or pCthr38. Since Corynebacterium glutamicum H-3149 (FERM BP-158) had a mutation of leucine-requirement, it was cultured on SSM medium supplemented with 200 μg/mℓ leucine, and treated with penicillin G. It was confirmed in the same manner as described above that the transformants contained each of the plasmids. The pChom3-carrying transformants were retransformed with pTS8, and thus, strains containing both pChom3 and pTS8 were obtained.

Isoleucine production tests of the transformants and their parent strains were carried out under the same conditions as in the foregoing section (5), provided that a production medium supplemented with 400 μg/mℓ leucine was used for Corynebacterium glutamicum H-3149 (FERM BP-158) and its transformants. Production test results are shown in Table 2.

Table 2

| Strain | Amount of isoleucine produced (g/ℓ) |
|---|---|
| Corynebacterium glutamicum K40 (FERM BP-455) | 1.2 |
| "      K40/pChom3 | 2.6 |
| "      K40/pTS8 | 1.8 |
| "      K40/pCthr38 | 4.2 |
| "      K40/pChom3, pTS8 | 3.9 |
| Brevibacterium fravum ATCC14067 | 0 |
| "      ATCC14067/pChom3 | 0.6 |
| "      ATCC14067/pCthr38 | 2.2 |
| "      ATCC14067/pChom3, pTS8 | 1.9 |
| Corynebacterium glutamicum H-3149 (FERM BP-158) | 0 |
| "      H-3149/pChom3 | 0.9 |
| "      H-3149/pCthr38 | 2.5 |
| "      H-3149/pChom3, pTS8 | 2.1 |

**Claims**

1. A process for producing L-threonine or L-isoleucine, which comprises transforming a microorganism belonging to the genus Corynebacterium or Brevibacterium with a recombinant DNA(s) wherein a DNA fragment(s) bearing genetic information responsible for the synthesis of homoserine dehydrogenase, homoserine kinase and threonine synthase originating from a microorganism belonging to the genus Corynebacterium or Brevibacterium is/are inserted, culturing the transformant in a culture medium until recoverable amounts of L-threonine or L-isoleucine are accumulated in the culture broth and recovering L-threonine or L-isoleucine therefrom.

2. A process according to Claim 1, wherein the genetic information responsible for the synthesis of homoserine dehydrogenase, homoserine kinase and threonine synthase is located in a single recombinant DNA or is separately located in independent recombinant DNAs.

3. A recombinant DNA containing a DNA fragment bearing genetic information responsible for the synthesis of threonine synthase originating from a microorganism belonging to the genus Corynebacterium or Brevibacterium.

4. A recombinant DNA containing a DNA fragment bearing all genetic information responsible for the synthesis of homoserine dehydrogenase, homoserine kinase and threonine synthase originating from a microorganism belonging to the genus Corynebacterium or Brevibacterium .

5. A microorganism belonging to the genus Corynebacterium or Brevibacterium which harbors a recombinant DNA containing a DNA fragment bearing genetic information responsible for the synthesis of threonine synthase originating from a microorganism belonging to the genus Corynebacterium or Brevibacterium.

6. A microorganism belonging to the genus Corynebacterium or Brevibacterium which harbors a recombinant DNA containing a DNA fragment bearing genetic information responsible for the synthesis of homoserine dehydrogenase, homoserine kinase and threonine synthase originating from a microorganism belonging to the genus Corynebacterium or Brevibacterium .

7. A microorganism according to Claim 6, wherein the genetic information is located in a single recombinant DNA or is separately located in independent recombinant DNAs.

8. A process for producing L-threonine or L-isoleucine, which comprises introducing a recombinant DNA(s) containing a DNA fragment(s) bearing genetic information responsible for the synthesis of homoserine dehydrogenase, homoserine kinase and threonine synthase originating from a microorganism belonging to the genus Corynebacterium or Brevibacterium into a microorganism belonging to the genus Corynebacterium or Brevibacterium and having an ability to produce L-lysine, culturing the resulting transformant in a culture medium until recoverable amounts of L-threonine or L-isoleucine are accumulated in the culture broth and recovering L-threonine or L-isoleucine therefrom.

# FIG. 1